# EUROPEAN PATENT APPLICATION

(11) **EP 4 289 436 A1**
(43) Date of publication of application: **13.12.2023**
(21) Application number: 22177882.2
(22) Date of filing: 08.06.2022
(51) Int. Cl.: A61K 31/713, C12N 15/113

(54) **PHARMACEUTICAL COMPOUND FOR PREVENTION OR TREATMENT OF LIVER FIBROSIS**

(71) Applicant: Medizinische Hochschule Hannover, 30625 Hannover (DE)
(72) Inventor: Sharma, PD Dr., Amar, 30625 Hannover (DE); Ott, Prof. Dr., Michael, 30625 Hannover (DE); Markovic, Jovana, 30625 Hannover (DE)
(74) Representative: Taruttis, Stefan Georg

(57) **Abstract**

The invention provides a compound, for use as single compound or for use as a combination of at least two compounds, for use in the prevention and/or treatment of liver fibrosis and/or liver cirrhosis, e.g. as a pharmaceutically active compound for use in the prevention or treatment of liver fibrosis, which compound is an inhibitor of hyaluronan synthetase, or an inhibitor of integrin alpha-6.

## Description

The present invention relates to pharmaceutical compounds, preferably to a combination of the pharmaceutical compounds for use in prevention and treatment of liver fibrosis and/or liver cirrhosis. Therein, the liver fibrosis can e.g. be pericentral or periportal liver fibrosis.

The compounds have been found to suppress the activation of quiescent hepatic stellate cells (qHSC), and hence suppress one of the key steps of fibrogenesis involving accumulation of extracellular matrix.

The activity of the pharmaceutical compounds for use in prevention and/or treatment of liver fibrosis and/or liver cirrhosis was shown in in vitro assays as well as in vivo in murine models of liver fibrosis and in the model of human liver bud.

### State of the art

Song et al., Cell Stem Cell 18, 797-808 (2016) described the generation of viral vectors AAV8 and AAV2p75.

### Object of the invention

It is an object of the invention to provide a compound suitable for use in prevention and treatment of liver fibrosis and/or liver cirrhosis. Preferably, the compound shall act on the pathway molecules that are involved in the transformation of healthy liver cells into myofibroblasts.

### Description of the invention

The invention attains the object by providing a compound, for use as single compound or for use as a combination of at least two compounds, for use in medical treatment, especially for use in the prevention and/or treatment of liver fibrosis and/or liver cirrhosis, e.g. as a pharmaceutically active compound for use in the prevention or treatment of liver fibrosis, which compound is an inhibitor of hyaluronan synthetase (HAS2, e.g. SEQ ID NO: 1 shows isoform Alpha-6X1X2B, identifier P23229-1 in UniProtKB), or an inhibitor of integrin alpha-6 (ITGA6, e.g. SEQ ID NO: 2 shows, identifier Q92819-1 in UniProtKB), or a combination of an inhibitor of hyaluronan synthetase (HAS2) and an inhibitor of integrin alpha-6 (ITGA6). The invention also provides an inhibitor of hyaluronan synthetase (HAS2), an inhibitor of integrin alpha-6 (ITGA6), and a combination of these, for use in medical treatment or for use in the prevention of diseases.

The inhibitor can be an inhibitor of expression of HAS2 or an inhibitor of expression of ITGA6, e.g. an inhibitory RNA, especially a microRNA (miR). Alternatively, the inhibitor can be a compound that inactivates HAS2 or ITGA6. An exemplary inhibitor of HAS2 for use in prevention and/or treatment of liver fibrosis and/or liver cirrhosis is 4-methylumbelliferone (4-MU or MU), 4-methylesculetin (ME), brefeldin A, or a combination of at least two of these.

It is assumed that an inhibitor of HAS2 and an inhibitor of ITGA6, singly and in combination, result in reduced formation of lower molecular weight hyaluronan (LMW-HA) from hyaluronan (HA), which is a high molecular weight polymer of the extracellular matrix and which is a biomarker for chronic liver fibrosis. The formation of HA in liver tissue is increased in liver diseases, and in liver injury, HA is degraded in liver tissue inter alia to LMW-HA, which LMW-HA can potentiate the injury by myofibroblast activation and inflammation. The inhibition of HAS2 has been found to result in a lower formation of HA, and accordingly to a lower formation of LMW-HA, and also in lower expression of fibrogenic marker proteins COL1A1, ACTA2, and S100A6 by liver tissue. Further, it is assumed that inhibition of ITGA6, e.g. in primary human myofibroblasts (MF), results in a lower level of membrane-associated AREG, e.g. by lower association of ITGA6 with AREG and subsequent less signalling by their associates. The AREG-ITGA6 associates are assumed to increase motility, invasion and activation of HSC, whereas the inhibition of ITGA6 results in lower levels of AREG-ITGA6 association, and hence in lower activation of HSC, and lower generation or activation of MF, e.g. determined as lower expression of fibrogenic markers COL1A1, ACTA2, and S100A6 in MF.

A preferred inhibitor is a microRNA, especially miR-296-3p (SEQ ID NO: 4) as an inhibitor of integrin alpha-6 (ITGA6), and miR-190b-5p (SEQ ID NO: 3) as an inhibitor of hyaluronan synthetase (HAS2).

The microRNA that forms the inhibitor can be expressed from an expression cassette encoding the microRNA, which expression cassette is introduced into liver cells, e.g. as a viral vector particle. Preferably, the coding sequence encoding the microRNA in the expression cassette is under the control of a promoter that is specifically expressed in pro-fibrotic myofibroblast cells (MF), which preferably is the Pdgfrb promoter (nucleotides 1 to 1346 of SEQ ID NO: 5). Alternatively, the microRNA can be expressed under the control of a constitutive promoter, e.g. the CMV promoter (nucleotides 381 to 584 of SEQ ID NO: 8), which is preferably preceded by a CMV enhancer, e.g. nucleotides 1 to 380 of SEQ ID NO: 8.

The expression cassette can be contained in a viral vector particle, e.g. on the basis of an adeno associated virus (AAV). An exemplary viral vector for producing miR-190b-5p can comprise the nucleic acid sequence of SEQ ID NO: 5 (AAV-Pdgfrb-miR-190b-5p), which contains an expression cassette for miR-190b-5p under the control of the Pdgfrb promoter. An exemplary viral vector for producing miR-296-3p can comprise the nucleic acid sequence of SEQ ID NO: 6 (AAV-Pdgfrb-296-3p), which contains an expression cassette for miR-296-3p under the control of the Pdgfrb promoter (nucleotides 1 to 1346 of SEQ ID NO: 6).

An exemplary viral vector for producing miR-296-3p can comprise the nucleic acid sequence of SEQ ID NO: 7 (AAV-Cmv-296-3p), which contains an expression cassette for miR-296-3p (nucleotides 656 to 809 of SEQ ID NO: 7) under the control of the CMV promoter (nucleotides No. 381 to 584 of SEQ ID NO: 7), which is preferably preceded by a CMV enhancer (nucleotides 1 to 380 of SEQ ID NO: 7). An exemplary viral vector for producing miR-190b-5p can comprise the nucleic acid sequence of SEQ ID NO: 8 (AAV-Cmv-miR-190b-5p), which contains an expression cassette for miR-190b-5p (nucleotides 656 to 807 of SEQ ID NO: 8) under the control of the CMV promoter.

In accordance with the efficacy of miR-190b-5p and miR-296-3p, single or in combination, the invention relates to miR-190b-5p and miR-296-3p, single or in combination, for use in the prevention or treatment of liver fibrosis, e.g. miR-190b-5p and miR-296-3p, single or in combination, as a pharmaceutically active compound for use in the prevention or treatment of liver fibrosis.

The inhibitor of hyaluronan synthetase or the inhibitor of integrin alpha-6, preferably their combination is suitable in a method of prevention or treatment of liver fibrosis.

The figures show in
- Fig. 1 micrographs with SMA immunocytochemical staining of primary human myofibroblasts after transfection with control miR (Scramble), with miR-190b-5p, or with miR-296-3p,
- Fig. 2 quantitative analysis of SMA staining of Fig. 1,
- Fig. 3 quantitative analysis of expression of ACTA2 in primary human myofibroblasts after transfection with control miR (Scramble), with miR-190b-5p, or with miR-296-3p,
- Fig. 4 graphs of levels of human miR-190b-5p and miR-296-3p,
- Fig. 5 a schematic of an assay,
- Fig. 6 expression levels of reporter dTomato,
- Fig. 7 schematic of in vivo administration of miRNAs in CC14-induced fibrosis model, and the expression levels of miR-190b-5p and miR-296-3p as determined by qRT-PCR,
- Fig. 8 expression levels of miR-190b-5p and miR-296-3p in livers of virally transduced animals,
- Fig. 9 expression levels of hydroxyproline in livers of virally transduced animals,
- Fig. 10 expression levels of Col1a1 and Acta2 in livers of virally transduced animals,
- Fig. 11 pictures and HE stainings of livers of virally transduced animals,
- Fig. 12 fibrosis area, levels of DEMIN and SMA in livers of virally transduced animals,
- Fig. 13 a schematic of in vivo administration of miRNAs in DDC-induced fibrosis model, and the expression levels of miR-190b-5p and miR-296-3p in livers of virally transduced animals,
- Fig. 14 levels of hydroxyproline in livers of virally transduced animals,
- Fig. 15 expression levels of Acta2, Fibrosis area, SMA levels and DEMIN levels in livers of virally transduced animals,
- Fig. 16 pictures, HE stainings, Sirius red stainings, SMA immunological stainings and DESMIN immunological stainings of livers of virally transduced animals,
- Fig. 17 flow cytometry results of HAS2 protein levels in miR-190b-5p or miR-296-3p transfected cells,
- Fig. 18 flow cytometry results of ITGA6 protein levels in miR-190b-5p or miR-296-3p transfected cells,
- Fig. 19 expression levels of luciferase reporter for HAS2 and ITGA6 in transduced primary human MF
- Fig. 20 stainings for HAS2 and ITGA6 in primary human MF transfected with miR-190b-5p1, miR-296-3p or Scramble,
- Fig. 21 expression levels of Has2 mRNA and of Itga6 mRNA in the liver tissue of mice transduced with respective AAVs in both pericentral and periportal liver fibrosis,
- Fig. 22 Western blots of protein levels of HAS2 and of ITGA6 in mice livers upon overexpression of miR-190b-5p and miR-296-3p,
- Fig. 23 immunohistochemistry analyses of formalin-fixed sections of human livers of different fibrosis stages for HAS2 and ITGA6,
- Fig. 24 A - E levels of TIMP1 and CTGF secretion as measured by ELISA and expression levels of fibrogenic markers ACTA2, COL1A1, and S100A6 in co-transfected human MF cells by qRT-PCR,
- Fig. 25 immunocytochemical stainings for SMA in transfected MF overexpressing ITGA6 or HAS2,
- Fig. 26 levels of LMW-HA in transfected primary human MF,
- Fig. 27 immunocytochemical stainings for SMA in MF treated with LMW-HA or AREG,
- Fig. 28 levels of membrane-associated AREG in transfected primary human MF,
- Fig. 29 expression levels of ACTA2 in transfected primary human MF,
- Fig. 30 expression levels of COL1A1 in transfected primary human MF,
- Fig. 31 expression levels of S100A6 in transfected primary human MF,
- Fig. 32 a schematic of an in vivo assay,
- Fig. 33 expression levels of miR-190b-5p and miR-296-3p in hepatic stellate cells of virally transduced animals,
- Fig. 34 expression levels of Has2 by RT-PCR in in hepatic stellate cells of livers of virally transduced animals,
- Fig. 35 expression levels of Itga6 by RT-PCR in in hepatic stellate cells of livers of virally transduced animals,
- Fig. 36 levels of fibrosis area, and of SMA in livers of virally transduced animals,
- Fig. 37 pictures, HE stainings, Sirius red stainings, SMA immunological stainings of livers of virally transduced animals,
- Fig. 38 Western blots of protein levels of HAS2 and of ITGA6 in mice livers upon overexpression of miR-190b-5p and miR-296-3p,
- Fig. 39 immunocytochemical stainings for ITGA6 or HAS2 in transfected MF overexpressing ITGA6 or HAS2,
- Fig. 40 a schematic of an in vitro assay using human liver buds,
- Fig. 41 expression levels of TIMP1 by ELISA in model human liver buds,
- Fig. 42 secretion levels of CCL2 by ELISA from model human liver buds,
- Fig. 43 expression levels of ALB in model human liver buds,
- Fig. 44 expression levels of HNF4A in model human liver buds,
- Fig. 45 expression levels of TTR in model human liver buds,
- Fig. 46 a schematic of an in vivo assay,
- Fig. 47 volcano plots of proteins of transduced fibrogenic MF from mouse livers,
- Fig. 48 volcano plots of proteins of transduced fibrogenic MF from mouse livers,
- Fig. 49 a schematic of an assumed signalling pathway,
- Fig. 50 relative expression levels of markers in MF cells and LX2 cells,
- Fig. 51 micrographs of MF cells and LX2 cells stained for SMA,
- Fig. 52A, 52B, 52C TIMP1 levels in three MF cultures in presence of miRNA mimics, and
- Fig. 53A, 53B, 53C CTGF expression levels.

The primers used for analyses of mouse genes in Sybr green qRT-PCR:

| Target genes | Forward Primer (5'-3') | Reverse Primer (5'-3') |
|---|---|---|
| *Acta2* | GTTCAGTGGTGCCTCTGTCA | ACTGGGACGACATGGAAAAG |
| | (SEQ ID NO: 9) | (SEQ ID NO: 10) |
| *Actb* | CTGTATTCCCCTCCATCG | AGTTGGTAACAATGCCATGT |
| | (SEQ ID NO: 11) | (SEQ ID NO: 12) |
| *Col1a1* | TAGGCCATTGTGTATGCAGC | ACATGTTCAGCTTTGTGGACC |
| | (SEQ ID NO: 13) | (SEQ ID NO: 14) |
| *Gapdh* | TGCCCCCATGTTTGTGATG | TGTGGTCATGAGCCCTTCC |
| | (SEQ ID NO: 15) | (SEQ ID NO: 16) |
| *Has2* | GAAAGGCAACCACAGCGAAG | ACATCAACCTACAASGCCGGG |
| | (SEQ ID NO: 17) | (SEQ ID NO: 18) |
| *Itga6* | CAGGTTCGAGTGACGGTGTT | CTCAGCCTTGTGATAGGTGGC |
| | (SEQ ID NO: 19) | (SEQ ID NO: 20) |
| *Rpl4* | CGCTGGTGGTTGAAGATA | CGGTTTCTCATTTTGCCC |
| | (SEQ ID NO: 21) | (SEQ ID NO: 22) |
| *S100a6* | TCCGTGTTTAGTTTTCCCTTCTGG | ATGTTCGTTTGCCTTGCCTC |
| | (SEQ ID NO: 23) | (SEQ ID NO: 24) |
| *Tgfbr1* | GTGGAAATCAACGGGATCAG | ACTTCCAACCCAGGTCCTTC |
| | (SEQ ID NO: 25) | (SEQ ID NO: 26) |

The primers used for analyses of human genes in Sybr green qRT-PCR:

| Target gene | Forward Primer (5'-3') | Reverse Primer (5'-3') |
|---|---|---|
| *HAS2* | TATCACTGCTGCTGGGACTTG | GGGCAAAACACTTTCAGGCG |
| | (SEQ ID NO: 27) | (SEQ ID NO: 28) |
| *ITGA6* | GAGCTTTTGTGATGGGCGATT | CTCTCCACCAACTTCATAAGGC |
| | (SEQ ID NO: 29) | (SEQ ID NO: 30) |
| *GAPDH* | TCTTCCAGGAGCGAGATCCCT | TGGTCATGAGTCCTTCCACGAT |
| | (SEQ ID NO: 31) | (SEQ ID NO: 32) |

In the figures, data are ±s.e.m, ^{∗} is P<0.05, ^{∗∗} is P<0.005, ^{∗∗∗} is P<0.0005. Scramble denotes an arbitrary miRNA species but excluding miR-296-3p and miR-190b-5p.

For qRT-PCR, total RNA was extracted with TRIzol reagent (Qiagen) according to the manufacturer's instructions. cDNAs were generated using TaqMan Reverse Transcription Reagents (ThermoFisher) or iScript cDNA kit (Bio Rad). For qRT-pCR, either TaqMan (Invitrogen) or SYBR Green (ThermoFisher) probes and kit were used. Relative mRNA expression was determined after expression normalization to GAPDH, gapdh, ACTB, actb or rpI4, as housekeeping genes. For miRNA reverse transcription and qRT-PCR, total RNAs were extracted with TRIzol reagent (Qiagen) according to the manufacturer's protocol. MiRNA's cDNAs were generated using TaqMan MiRNA Reverse Transcription Reagents (ThermoFisher) and miRNA-specific RT primers. Relative miRNA expression was determined after normalization to U6 or sno.

Viral titer was determined by quantitative polymerase chain reaction (qRT-PCR) using primers targeting miRNAs or Pdgfrb. Primary murine HSC isolation was performed according to Maschmeyer et al., J Vis Exp 2710 (2011). In short, mice livers were perfused with Pronase E solution and Collagenase P solution at a flow speed of 6.5 mL/min for 4.5 min at 37°C. All perfused mouse livers were collected and digested in vitro with 50 ml Pronase E-Collagenase P solution and 1 ml DNase I at 37°C for 20 min. The cell suspension was filtered through 100 µm cell strainers and centrifuged at 600 × g at 4°C for 10 min. After discarding the supernatant, the cells were resuspended in Gey's Balanced Salt Solution-B (GBSS-B) and 150 µL DNase 1 solution was added to the cell suspension. The cell suspension was centrifuged at 600 × g at 4°C for 10 min. After carefully discarding the supernatant, the cells were suspended with 36 ml GBSS-B buffer and 150 µL DNase 1 solution was added to the solution. 14 ml Nycodenz solution (8 g Nycodenz with 28 ml GBSS-A buffer) was added to the cell suspension to reach an 8% Nycodenz gradient. The gradients were centrifuged at 1500 × g at 4°C for 15 min without breaking. Primary HSCs were harvested from the interphase of the gradients.

For flow cytometry, antibodies specific for ITGA6 (Invitrogen, 25-0495-82), for HAS2 (sc-514737 AF488) and for AREG (sc-74501) were used. Prior to staining of ITGA6 and of AREG, cells were fixed with 4% PFA, while HAS2 staining was performed on fixed and permeabilized cells (4% PFA/0.5% Tween20).

Blood was collected in the retroorbital manner and sera was prepared and stored at -20°C. AST, ALT, and bilirubin were measured in the sera. TIMP1 and CCL2 (Abcam), CTGF (Abbexa), AREG (SigmaAldrich), LMW-HA (R&D Systems) were quantified according to the manufacturer's protocol.

For histology, immunohistochemistry, and immunofluorescence, liver tissues were fixed with 4% formalin, embedded in paraffin, and cut into 2-5 µm thick sections. For Sirius Red staining, after deparaffinization, sections were stained with solution of Direct Red 80 (Merck) in picric acid (Merck) for 1 h at room temperature. After that, sections were washed in acetic water and slides were dehydrated and mounted. Immunohistochemistry for HAS2 (Invitrogen, PA5-115388), SMA (ab5694) and DESMIN (ab15200) were performed using the Vectastain ABC-HRP (Biozol, VEC-PK-6101) kit according to the manufacturer's instructions. ITGA6 immunohistochemical staining was performed with the use of ITGA6 antibody (Invitrogen, 14-0495-82). Quantification of immunohistochemical stainings were performed with ImageJ software.

For HAS2 and ITGA6 Western blots, proteins were isolated from liver tissues with Mem-PERTM Plus Membrane Protein Extraction Kit (Thermo Scientific) according to the manufacturer's instructions. Briefly, 20-40 mg of livers were washed with 4 mL of Cell Wash Solution. The wash was discarded, and 1 mL of permeabilization buffer was added to the tissue. Tissue was homogenized and the mixture was incubated for 10 min on ice with constant mixing. The mixture was then centrifuged at 16000 × g for 15 min at 4°C. The supernatant containing cytosolic proteins was transferred to a new tube and kept on ice. The pellet was resuspended in 1 mL of solubilization buffer. The mixture was homogenized via pipetting and incubated for 30 min at 4°C with constant mixing. The mixture was centrifuged at 16000 × g for 15 min at 4°C. The supernatant containing membrane-associated proteins was transferred to a new tube and kept on ice. Fraction containing soluble proteins and fraction containing membrane-associated proteins were mixed in a 1:1 ratio, and the mixture was used for analyses.

Liver biopsy specimens were obtained by liver biopsies performed percutaneously under local anesthesia. An approximately 5 mm portion of the needle biopsy was immediately preserved in Allprotect Tissue Reagent (QIAGEN) and then stored at -80° C. The remaining cylinder was fixed in 4% neutral buffered formalin and embedded in paraffin wax. Histological examination and scoring for fibrosis stage (Ishak score) were performed by experienced liver pathologists.

### Example 1: Antifibrotic activity of miR-296-3p and miR-190b-5p in MF

Primary human myofibroblasts (MF, purchased from Innoprot (Bizkaia, Spain)) that were used in the in vitro assays were assed for their expression of known fibrogenic markers DES, ACTA2, LOX2, and COL1A in comparison to SMALX2 cells (LX2), which is an established human HSCs cell line. 96-well-plates (TRP) were coated with gelatin at 37°C for 10 minutes. After discarding the gelatin solution, 9600 primary human myofibrobiasts were seeded and cultured in Dulbecco's modified Eagle's medium (DMEM) supplemented with 10% heat-inactivated fetal bovine serum, 1% penicillin-streptomycin, 2-mercaptoethanol and nonessential amino acids.

The results indicate significantly higher expression of all these markers in MF than in SMALX2 cells. Additionally, immunocytochemical staining of alpha smooth muscle actin (SMA) confirmed the pro-fibrogenic profile of MF.

The anti-fibrotic potential of miRNA was evaluated independently for separate batches of primary human MF. Based on the modulation of TIMP1 secretion and measurement of the levels of connective tissue growth factor (CTGF) in the culture medium collected from MF transfected with comparative miRNAs, the miRNAs miR-296-3p which is an inhibitor of integrin alpha-6 (ITGA6), and miR-190b-5p which is an inhibitor of hyaluronan synthetase were identified. Additionally, the secretion of CTGF, also known as CCN2, which is a member of CCN family of extracellular matrix-associated heparin-binding proteins that acts as an agonist of the TGF-β signalling pathway, which is one of the most potent pathways in fibrosis, was evaluated in culture supernatants of miRNA transfected myofibroblasts, using scramble transfected cells as control in a CTGF ELISA. Also the analysis of secretion of CTGF confirmed that hsa-miR-190b-5p and hsa-miR-296-3p significantly and consistently regulated CTGF secretion in all three batches of primary cells, showing the anti-fibrotic activity of these miRNAs.

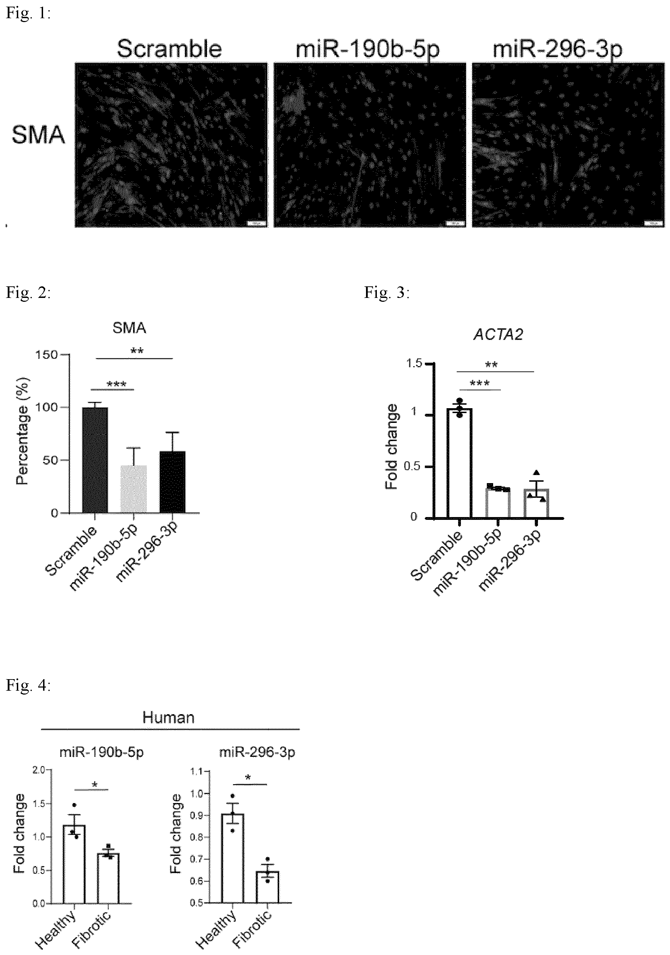

The anti-fibrotic activity of miR-296-3p and miR-190b-5p was confirmed in SMA immunocytochemical staining on MF transfected with scramble, with miR-190b-5p, or with miR-296-3p, the micrograph is depicted in Fig. 1. These miRNAs significantly decreased SMA levels, compared with the control (Scramble).

The expression of platelet-derived growth factor receptor beta (PDGFRb) on the surface of MF transfected with miR-190b-5p, miR-296-3p, or scramble (control). PDGFRb is an additional characteristic fibrogenic marker whose expression is upregulated on the surface of activated MF2122 was determined by FACS. Surface expression of PDGFRb was observed to be decreased upon transfection with miR-190b-5p to 22%, upon transfection with miR-296-3p decreased to 28.5%, and in the scramble control to only 58%. This shows that presence of miR-190b-5p and miR-296-3p resulted in a significantly stronger decrease of surface expression of PDGFRb.

The qRT-PCR analyses, data depicted in Fig. 3, showed lower expression of LOX2 and ACTA2 in MF transfected with miR-190b-5p or miR-296-3p, compared with the control transfected cells.

Further, expression of both miR-190b-5p and miR-296-3p was analyzed by qPCR in fibrosis stage 3 (Ishak score) human fibrotic liver biopsies (Fig. 4, Fibrotic) and in human healthy liver tissue (Fig. 4, Healthy). The expression of both miR-190b-5p and miR-296-3p was significantly downregulated in the fibrotic tissues than in the non-fibrotic ones.

These results identify hsa-miR-296-3p and hsa-miR-190b-5p as fibrogenic regulators.

### Example 2: In vivo inhibition of HAS2 or ITGA6 for treatment of liver fibrosis

As an exemplary inhibitor of HAS2 miR-190b-5p was used, miR-296-3p was used as an exemplary inhibitor of ITGA6.

Since primary human MF are considered as the foremost contributors to liver fibrosis, miR-190b-5p and miR-296-3p were transfected preferentially into myofibroblasts in vivo. For preferential expression in MF, the most abundant cells in the liver, which are hepatocytes, were circumvented by expressing the miR-190b-5p or miR-296-3p under the control of the MF-specific Pdgfrb promoter in vivo. Upregulation in the activity of Pdgfrb promoter has been previously reported upon activation of HSC23. The coding sequence for miR-190b-5p or miR-296-3p was cloned downstream of Pdgfrb promoter in an adeno associated virus (AAV) vector. AAV8-Pdgfrb-296-3p (SEQ ID NO: 6) contains the expression cassette for miR296-3p under the control of the Pdgfrb promoter, AAV8-Pdgfrb190b-5p (SEQ ID NO: 5) contains the expression cassette for miR-190b-5p under the control of the Pdgfrb promoter. Expression of miR-190b-5p or miR-296-3p in myofibroblasts after AAV administration in vivo was analyzed after intravenously administrating AAV-control, AAV8-Pdgfrb190b-5p or AAV8-Pdgfrb-296-3p into Lrat-Cre-dTomato mice (8-12-week-old male BALB/c mice). In this mouse model, CredTomato expression is driven by the lecithin retinol acyltransferase (trat) promoter, which is preferentially active in HSC and HSC-derived MF. AAV8 and AAV2p75 were produced as described by Song et al., Cell Stem Cell 18, 797-808 (2016).

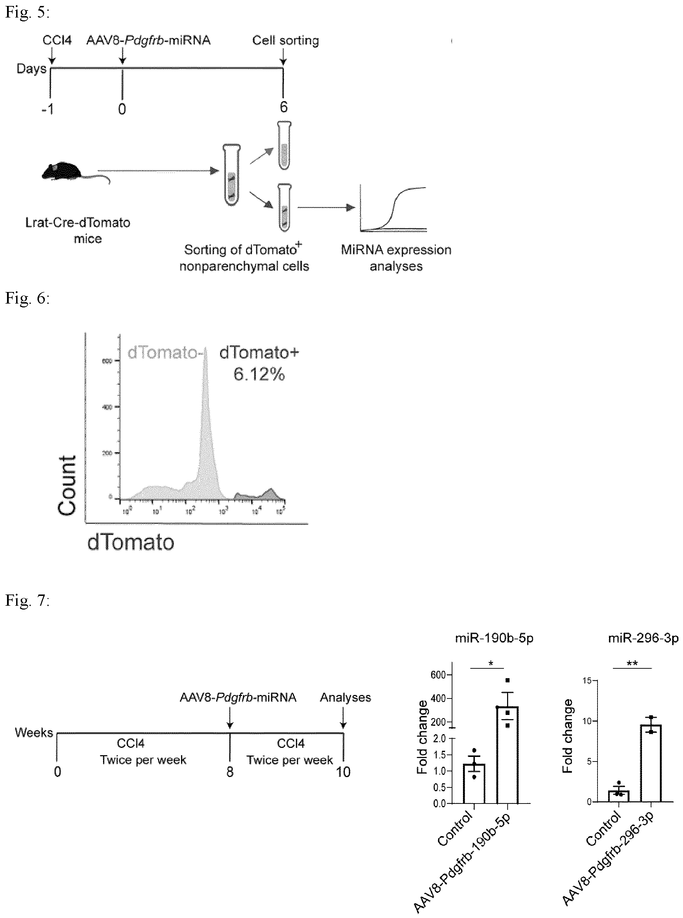

As schematically depicted in Fig. 5, prior to the AAV8 injections, mice were treated with a single dosage of 8 µ1/g 10% CCl₄/olive oil. Six days after AAV8 administrations, hepatocytes and all non-parenchymal cells were isolated, among which dTomato positive (HSC and HSC-derived MF) and dTomato negative cell populations were sorted. Fig. 6 depicts the result that dTomato positive cells (dTomato+) were detected to 6.12%, with the remainder to 100% being dTomato negative cells (dTomato-). In dTomato positive cell populations, the expression of miR-190b5p and miR-296-3p was determined by qRT-PCR, results depicted in Fig. 7. The expression of both miRNAs was significantly higher in dTomato positive cells isolated from mice injected with respective AAV8-PdgfrbmiRNA, than in the control mice. These data in Lrat-Cre-dTomato mice provide evidence that delivery of AAV8-Pdgfrb-190b-5p or AAV8-Pdgfrb-296-3p results in upregulation of the respective miRNA in HSC and HSC-derived MF.

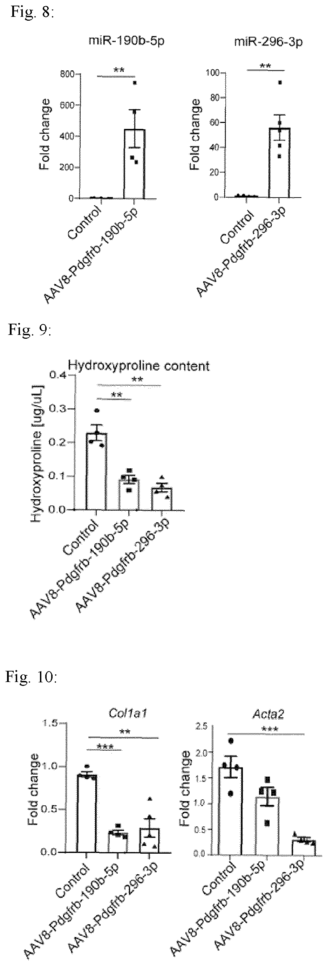

The overexpression of miR-190b-5p and miR-296-3p in myofibroblasts was found to ameliorate pericentral liver fibrosis when analysing the anti-fibrotic activity of miR-190b-5p and/or of miR-296-3p in vivo. A murine model of pericentral liver fibrosis was induced by injecting CCl₄ bi-weekly for 8 weeks. At the 8th week of CCl₄ injections, one of miR-190b-5p and miR-296-3p was overexpressed using high titer adeno-associated virus (AAV) vector serotype 8 (1×10¹⁰ vector particles) encoding either miR190b-5p or miR-296-3p under the control of Pdgfrb promoter. CCl₄ administration was continued for 2 more weeks after AAV injections, after which mice were sacrificed. Overexpression of each of the miRNAs in the liver tissue was confirmed by qRT-PCR as depicted in Fig. 8, showing expression levels.

Hydroxyproline content in the livers was measured as an unbiased parameter that correlates with the amount of collagen in tissue. The results are depicted in Fig. 9, showing a reduced concentration of hydroxyproline in the livers of mice injected with AAV8-Pdgfrb-190b-5p or AAV8-Pdgfrb-2963p, compared with the control mice. Additionally, the expression levels of fibrogenic genes Col1a1 and Acta2 were measured. Both Col1a1 and Acta2 were significantly downregulated upon overexpression of miR-190b-5p or miR-296-3p, as depicted in Fig. 10.

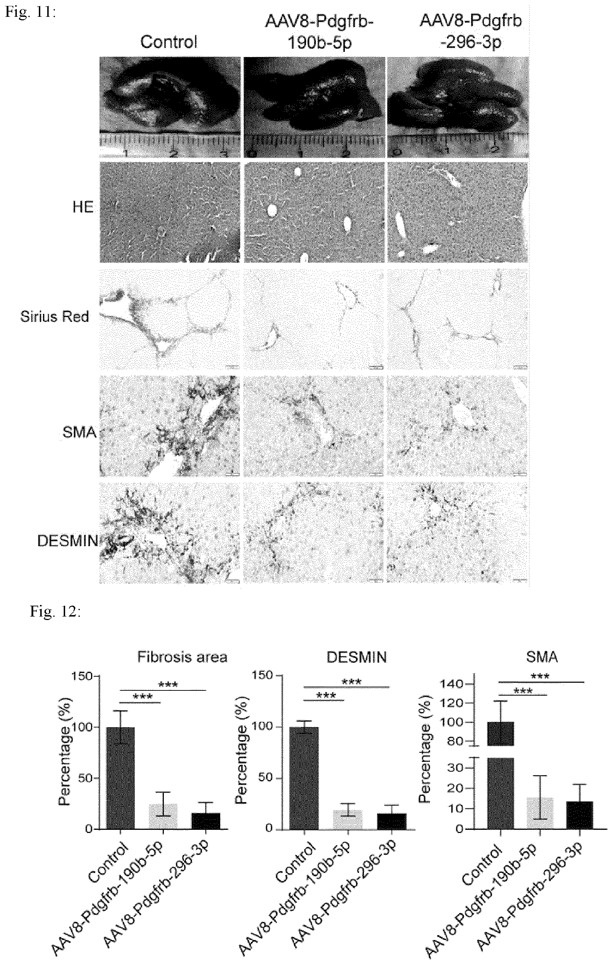

The degree of fibrosis was determined by HE and Sirius Red staining and SMA and DESMIN staining, as shown in Fig. 11 for control liver and livers transduced with AAV8-Pdgfrb-190b-5p or AAV8-Pdgfrb-2963p. Histological analyses and SMA and DESMIN immunohistochemistry indicated mitigation of liver fibrosis in both AAV8-Pdgfrb-190b-5p and AAV8-Pdgfrb-296-3p groups of mice, compared with the control group, as depicted in Fig. 12.

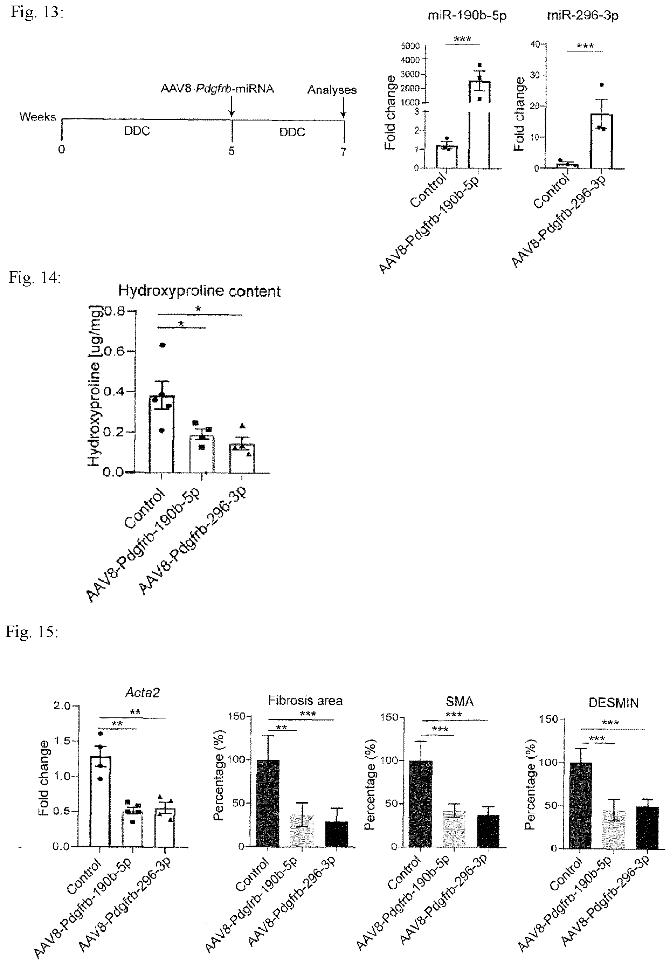

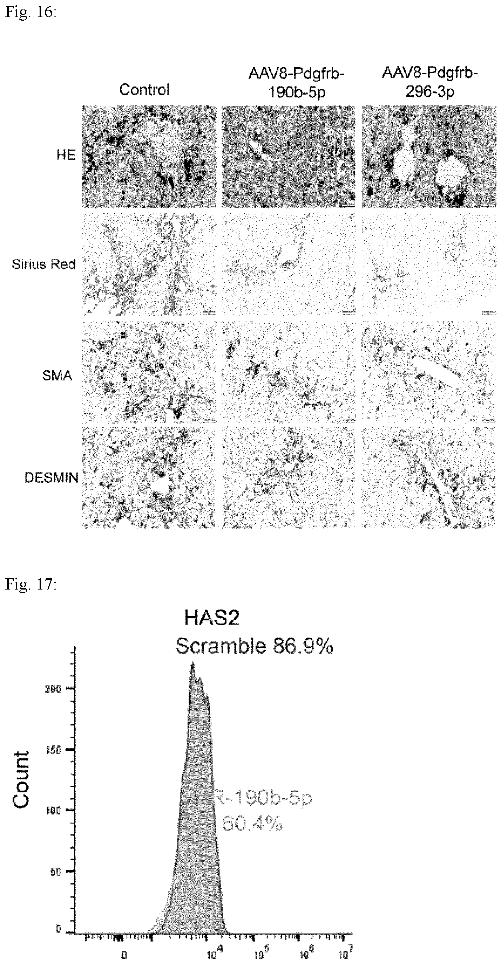

In addition, ALT, AST, bilirubin, and albumin levels were measured in the sera collected from mice injected with control virus, with AAV8Pdgfrb-190b-5p or with AAV8-Pdgfrb-296-3p. Fig. 13 shows the results that indicate that AAV8-mediated overexpression of miR-190b-5p or of miR-296-3p in myofibroblasts ameliorates CCl₄-induced pericentral liver fibrosis. Furthermore, the AAV8 viral vectors were produced containing the coding sequences for miR-190b-5p (AAV8-Cmv-190b-5p) or miR-296-3p (AAV8-Cmv-296-3p) that were cloned downstream of the constitutively active CMV promoter. These AAV viral vectors were administrated at the 8th week of the CCl₄ injections, and two weeks upon the AAV administration, the mice were sacrificed for analyses. Fig. 14 depicts the data of qRT-PCR confirming overexpression of these miRNAs from the AAV vectors in the collected liver tissues. Afterwards, hydroxyproline content was found decreased in livers overexpressing miR-190b-5p (AAV8-Cmv-190b-5p) or miR-296-3p (AAV8-Cmv-296-3p) as depicted in Fig. 15. Measurement of levels of fibrogenic markers Col1a1 and Acta2 (Fig. 16) showed decrease of both Col1a1 and of Acta2 in livers overexpressing miR-190b-5p (AAV8-Cmv-190b-5p) or miR-296-3p (AAV8-Cmv-296-3p).

The extent of liver fibrosis was determined via HE and Sirius Red staining, as well as SMA and DESMIN staining as shown in Fig. 17. The data indicate mitigation of liver fibrosis in both AAV8-Cmv-190b-5p and AAV8-Cmv-296-3p groups, when compared with the control group. Taken together, CMV-driven overexpression of miR-190b-5p and miR-296-3p ameliorates CCl₄-induced liver fibrosis.

### Example 3: In vivo inhibition of HAS2 or ITGA6 for treatment of liver fibrosis

As an exemplary inhibitor of HAS2 miR-190b-5p was used, miR-296-3p was used as an exemplary inhibitor of ITGA6 for use in the treatment of periportal liver fibrosis. It was found that overexpression of miR-190b-5p and miR-296-3p mitigates periportal liver fibrosis.

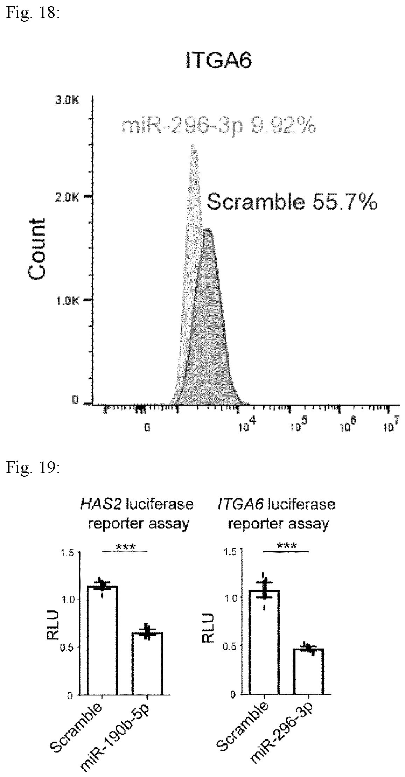

As a model, liver fibrosis was induced by feeding mice with a 3,5-diethoxycarbony1-1,4-dihydrocollidine (DDC)- containing diet for 5 weeks. At the 5th week, AAV control, AAV8-Pdgfrb-190b-5p or AAV8Pdgfrb-296-3p were administrated intravenously. Mice were sacrificed two weeks afterwards, during which DDC-containing diet was continued. Since the degree of fibrosis is higher in this model, than in the pericentral model of Example 2, it was sought to address whether the AAV8-Pdgfrb system enables the expression of miRNAs in myofibroblasts preferentially in this model as well. Myofibroblasts were isolated from the injected mice via in vivo perfusion. Overexpression of miR-190b-5p and miR296-3p in the myofibroblasts isolated from the mice injected with respective AAVs was confirmed as shown by the data of Fig. 18. In addition, the expression of Acta2 and Desmin in the isolated cells was measured by qRT-PCR, data shown in Fig. 19. The data show that even in the cholestasis-induced liver fibrosis, AAV8-Pdgfrb-miRNA enters and attains the overexpression of miRNAs in myofibroblasts, resulting in inhibition of HAS2 and ITGA6.

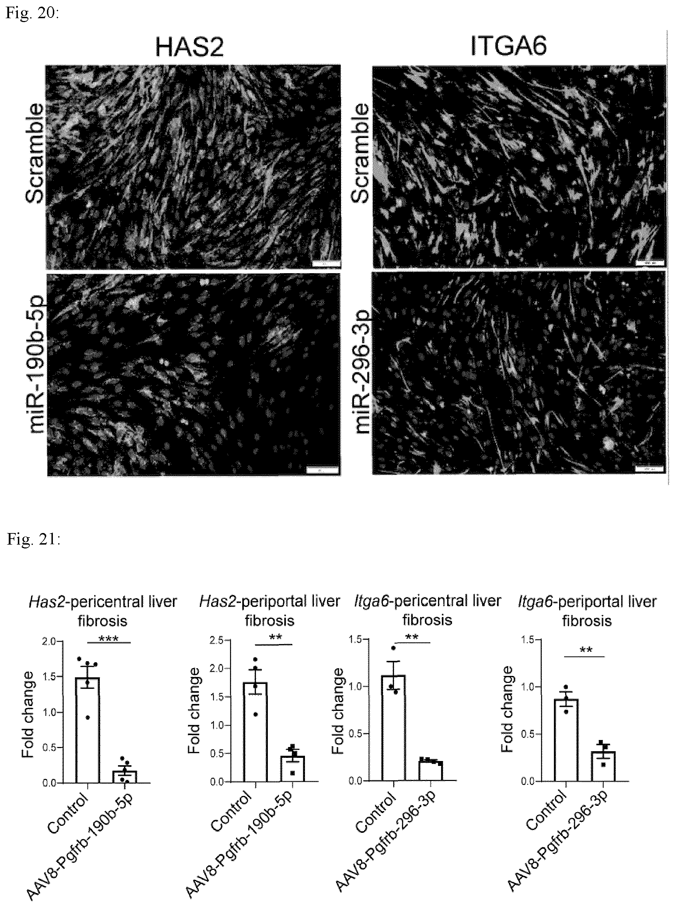

Moreover, the overexpression of miR-190b-5p and miR-296-3p supresses the levels of fibrogenic markers in myofibroblasts. The extent of fibrosis in the liver tissues was analysed by measuring the hydroxyproline content in the liver tissues of mice injected with AAV-control, AAV8-Pdgfrb190b-5p or AAV8-Pdgfrb-296-3p, results depicted in Fig. 20 show decrease of hydroxyproline by miR-190b-5p or miR-296-3p.

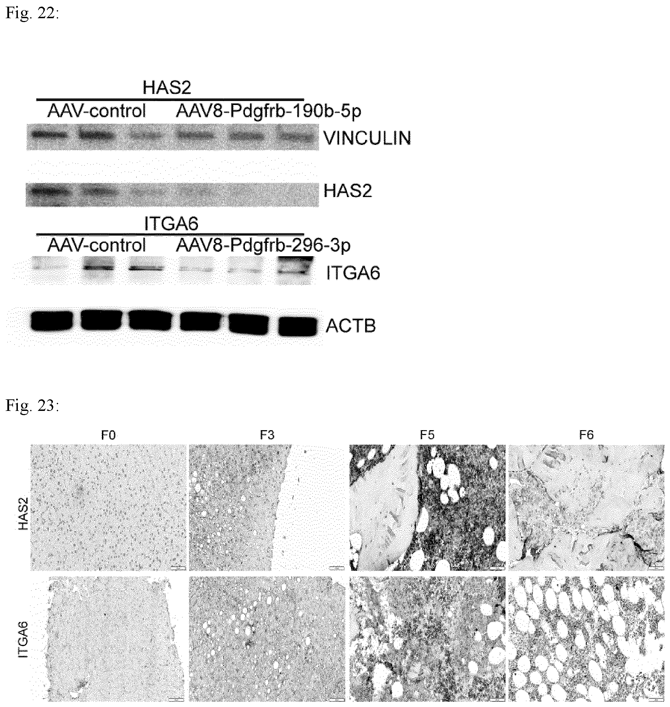

Analyses of the expression of fibrogenic markers Tgfbrl and Acta2 by qRT-PCR (Fig. 21) shows that both, the total collagen content, as determined by hydroxyproline assay, and mRNA levels of the fibrogenic markers were significantly downregulated upon overexpression of miR-190b-5p or miR-296-3p, compared with the control. The anti-fibrotic activity of these miRNAs was further confirmed with HE and Sirius red staining (Fig. 22, Fig. 23).

In addition, significantly less SMA and DESMIN positive cells was observed in the liver sections of mice injected with AAV8-Pdgfrb-190b-5p or AAV8-Pdgfrb296-3p, compared with the control group (Fig. 22, Fig. 23). These results show that inhibitors of HAS2 or ITGA6, exemplified by overexpression of miR-190b-5p and miR-296-3p, in MF dampens periportal liver fibrosis.

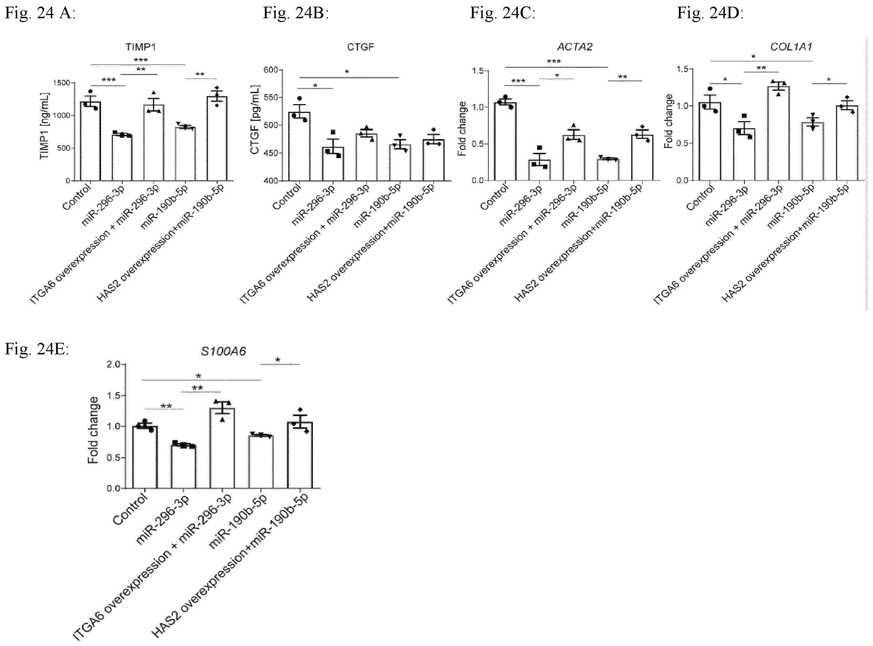

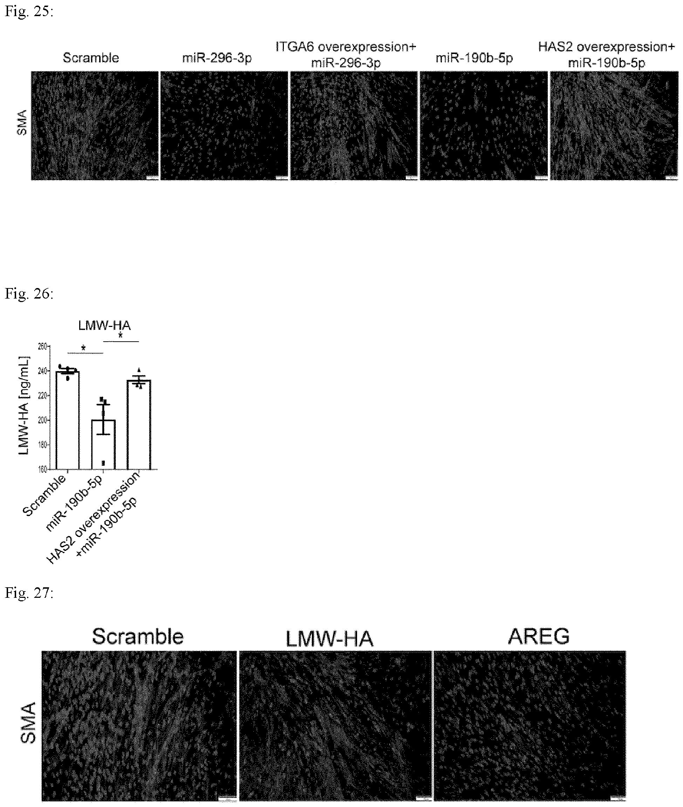

Also, CMV-driven overexpression of miR-190b-5p and miR296-3p was found to mitigate periportal liver fibrosis. Therein, at the 5th week of the DDC-containing diet, AAV control, AAV8-Cmv-190b-5p or AAV8-Cmv-296-3p was administrated intravenously. The DDC containing diet was continued for 2 more weeks, after which mice were sacrificed. Confirmation of overexpression of these miRNAs in the liver tissues was by qRT-PCR (Fig. 24). Determination of hydroxyproline content (Fig. 25), as well as the expression of fibrogenic markers Tgfbrl and Acta2 (Fig. 26) also showed anti-fibrotic activity of miR-190b-5p and miR296-3p.

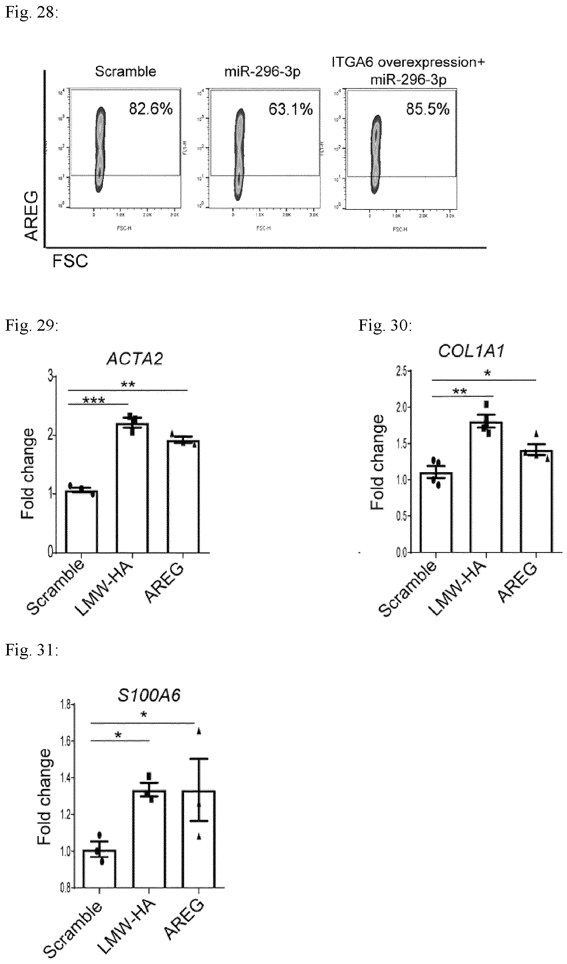

HE, Sirius red staining, as well as SMA and DESMIN immunohistochemical stainings (Fig. 27, Fig. 28) confirmed anti-fibrotic activity of these inhibitors of HAS2 and ITGA6.

Based on the hydroxyproline content, the expression of fibrogenic markers, and immunohistochemical staining the data show that AAV8-Cmv-190b-5p or AAV8-Cmv-296-3p administration leads to amelioration of periportal liver fibrosis, compared with the control group.

### Example 4: Analyses of the mechanism of action of inhibition of HAS2 and ITGA6

Hyaluronan synthase 2 (HAS2) and integrin alpha-6 (ITGA6) were identified as targets of miR-190b-5p and miR-296-3p, respectively, when analysing posttranscriptional regulation of HAS2 by miR-190b-5p, and of ITGA6 by miR-296-3p. For example, gene set enrichment analyses (GSEA) indicated downregulation of several signalling pathways in miRNA modulated groups, compared with scramble. Among the downregulated signalling pathways are TGFbeta receptor complex signalling, NGF signalling, and growth hormone receptor signalling.

The anti-fibrotic mechanism of miR-190b-5p and miR-296-3p, respectively, by downregulation or inhibition of HAS2 and ITGA6 was confirmed by in silico analyses showing that 3' UTRs of HAS2 and ITGA6 contain binding sites for miR190b-5p and miR-296-3p.

Also, measurements of protein levels of HAS2 and ITGA6 in scramble, miR-190b-5p or miR-296-3p transfected primary human MF via flow cytometry showed that both, HAS2 and ITGA6 protein levels were decreased in miR-190b-5p or miR-296-3p transfected cells, compared with scramble transfected cells, data shown in Fig. 29 for HAS2 and Fig. 30 for ITGA6.

Cloning of the human 3'UTRs of HAS2/ ITGA6 into a dual luciferase pmiRGLO vector and performing a luciferase reporter assay confirmed downregulation by miR-190b-5p or miR-296-3p. For the luciferase reporter assay 3'UTRs of ITGA6 and of HAS2 were amplified by PCR from HEK293 gDNA. The amplicons were cloned into a miRGLO vector (Promega). Primary human myofibrobiasts were transfected with 100ng miRGLO-3'UTR plasmid and 25 nM miRNA. 48h later, dual-GLO luciferase reagent was added and incubated for 45 min before measuring the firefly luminescence. Subsequently, StopGLO reagent was added and incubated for 30 min before reading the renilla luminescence. Luminescence intensity was calculated using the firefly/renilla ratio. Primary human MF were co-transfected with pmiRGLO-HAS2 and miR-190b-5p1 Scramble, or pmiRGLO-ITGA6 and miR-296-3p1 Scramble. The resulting data depicted in Fig. 31 by the reduced expression of luciferase reporter show that miR-190b-5p binds to the HAS2 3'UTR, while miR-296-3p binds to the ITGA6 3'UTR.

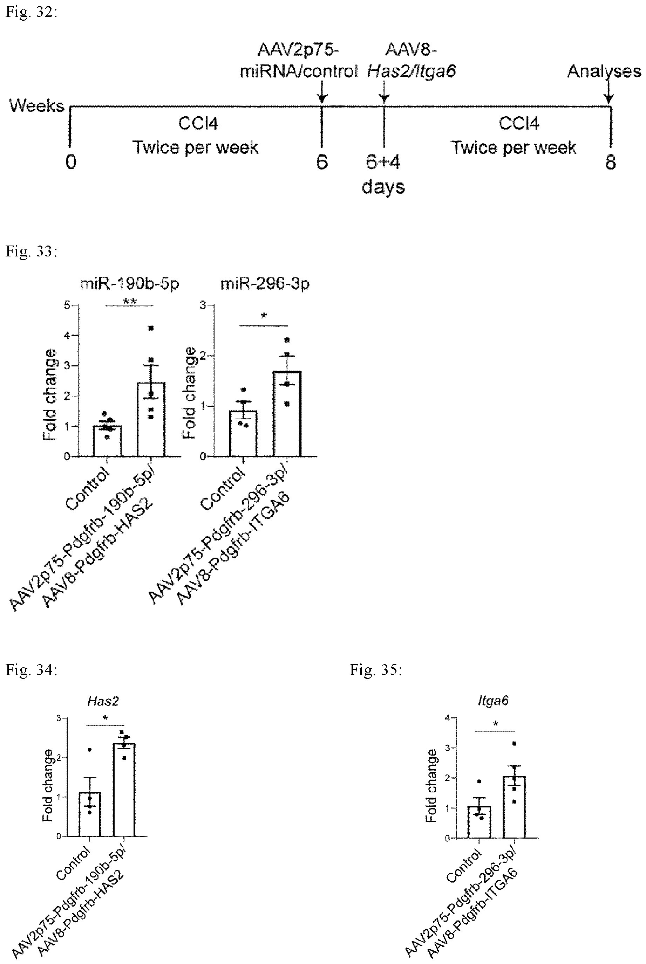

Additionally, downregulation of expression of HAS2 and ITGA6 by miR-190b-5p and miR-296-3p was further confirmed by immunocytochemistry, depicted in Fig. 32, showing that overexpression of miR190b-5p drastically reduced expression of HAS2, and overexpression of miR296-3p drastically reduced expression of ITGA6, compared to overexpression of scramble (control).

Taken together, it was found that miR-190b-5p and miR-296-3p seed sequences bind with the 3'UTRs of HAS2 and ITGA6 in vitro, which results in lower protein levels of HAS2 and ITGA6. HAS2 was identified as target of miR-190b-5p, and ITGA6 was identified as target of miR-296-3p, identifying an inhibitor of HAS2 and/or of ITGA6 for use in the treatment of liver fibrosis.

The target genes HAS2 and ITGA6 have been found to be downregulated in vivo by AAV8-Pdgfrb-190b-5p or AAV8-Pdgfrb-296-3p administration. Measurements by qRT-PCR of Has2 mRNA and of Itga6 mRNA levels in the liver tissue of mice injected with respective AAVs in both pericentral and periportal mode of liver fibrosis showed strong downregulation, results are depicted in Fig. 33. The mRNA levels of Has2 and of Itga6 were lower in miRNA modulated groups than in the control group. Also, analyses of protein levels of HAS2 and of ITGA6 by Western blotting (Fig. 34) and immunohistochemistry (Fig. 35) showed that HAS2 and ITGA6 protein levels were lower in vivo in mice livers upon overexpression of miR-190b-5p and miR-296-3p, respectively, compared with control mice.

Moreover, it was confirmed by immunohistochemistry that these targets in the absence of their inhibitors are mainly expressed in MF in human fibrotic liver biopsies, as shown in Fig. 35, wherein formalin-fixed sections of livers, without fibrosis (FO), fibrosis stage 3 (F3), fibrosis stage 5 (F5), or fibrosis stage 6 (F6) were immunostained for HAS2 or ITGA6. The data show low expression of both HAS2 and ITGA6 in FO, and increasing expression of HAS2 and ITGA6 with the progression of fibrosis to F3 and F5. Interestingly, in F6 the expression of HAS2 and ITGA6 is lower than the one observed in F5.

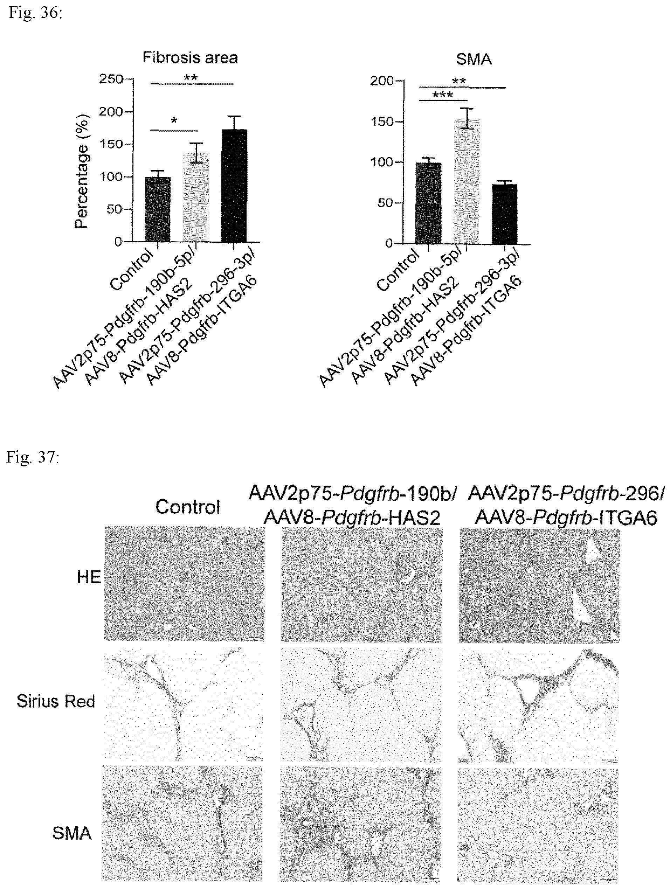

Further, overexpression of ITGA6 and HAS2 in myofibroblasts in vitro showed that these are relevant in development of liver fibrosis, as overexpression of HAS2 and ITGA6 restored the pro-fibrogenic activity of myofibroblasts. Primary human MF were co-transfected with miR-190b-5p and HAS2 expressing vector (pCMV6-XL6-HAS2), or with miR-296-3p and ITGA6 expressing vector (pCMV6-XL4-ITGA6), wherein both pCMV6-XL6-HAS2 and pCMV6-XL4-ITGA6 do not contain the 3'UTR sequences. 48h after transfection the culture medium was collected, in which TIMP1 and CTGF secretion was measured by ELISA. Additionally, the levels of fibrogenic markers ACTA2, COL1A1, and S100A6 in the co-transfected cells was assessed by qRT-PCR (Fig. 36A - E). TIMP1 levels were significantly higher in the cells co-transfected with miR-190b5p/HAS2 or co-transfected miR-296-3p/ITGA6 than in the cells only transfected with miR-190b-5p or miR-296-3p. The mRNA levels of fibrogenic markers and SMA protein levels were also higher in the co-transfected cell (Fig. 37).

Taken together, these results show that HAS2 and ITGA6 contribute to the activation of MF and to their profibrogenic profile, and that inhibition of HAS2 and/or of ITGA6 reduce the development of liver fibrosis.

HAS2 is an enzyme that is essential for the synthesis of hyaluronan. Besides, it also regulates cellular migration, proliferation and transdifferentiation. Hyaluronan (HA) is a high molecular weight polymer that is a biomarker for chronic liver fibrosis. Its formation is increased in liver diseases, and it is a component of ECM. In liver injury, it is degraded to lower molecular weight hyaluronan (LMW-HA) and medium molecular weight (MMW-HA) hyaluronan. LMW-HA is formed enzymatically or non-enzymatically and it further potentiates the injury via myofibroblast activation and inflammation.

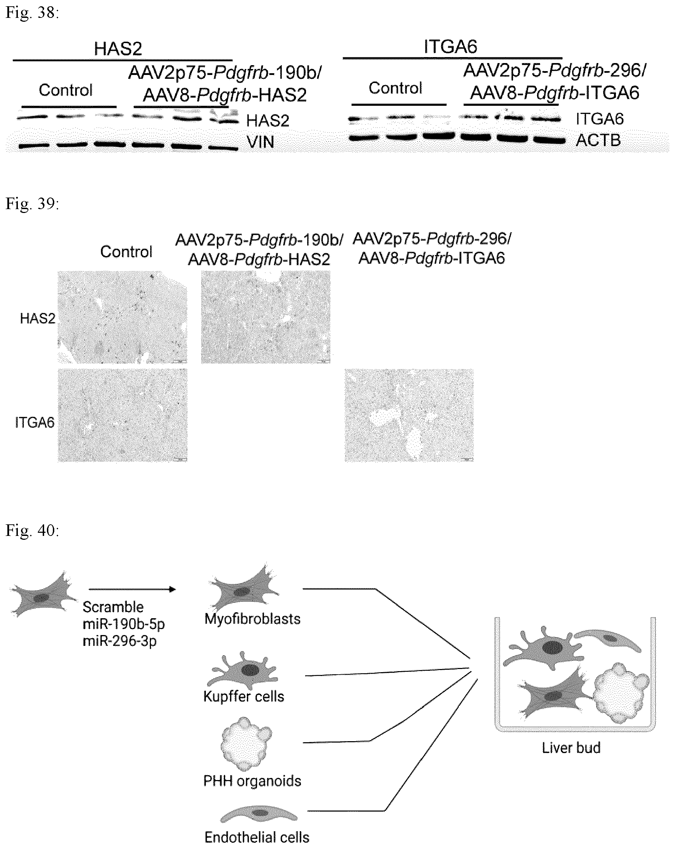

Currently, the mechanism of action of HAS2 and ITGA6 in aiding to fibrogenesis is assumed to be reduced via miR-190b-5p mediated downregulation of HAS2, resulting in lower formation of HA and therefore lower amount of LMW-HA. To validate this hypothesis, LMW-HA was measured in the culture medium collected from primary human MF transfected with miR-190b-5p or scramble, or co-transfected with miR-190b and pCMV6-XL6-HAS2 plasmid (Fig. 38). There is a significant decrease in the amount of LMW-HA upon miR-190b-5p transfection, which is restored upon HAS2 overexpression. As an additional validation, primary human MF were treated with 100 pg/mL LMW-HA for 48h upon which COL1A1, ACTA2 and S100A6 were measured by qRT-PCR. The treatment of cells with LMW-HA increased the expression of fibrogenic markers, as well as increasing SMA protein levels as indicated by immunocytochemistry (Fig. 39). Thus it was found that miR-190b-5p-mediated inhibition of HAS2 leads to amelioration of liver fibrosis due to lower formation of LMW-HA.

Amphireguline (AREG), a protein which has been previously identified as an important regulator of liver fibrosis, was reported to interact with ITGA6. AREG is synthesized as a transmembrane protein (Pro-AREG), which is then cleaved into AREG that binds to EGFR, among others. Binding of AREG leads to the formation and release of pro-fibrogenic and proinflammatory cytokines, such as TGFβ and CCL239A. It is therefore assumed that the interaction of AREG with ITGA6 is an important pathway that regulates motility, invasion and activation of HSC. AREG binds to the membrane-located ITGA6, which participates in HSC activation. Measurement of levels of membrane-associated AREG in primary human MF transfected with miR-296-3p or scramble, or co-transfected with miR-296-3p and ITGA6 expressing plasmids, showed that in miR-2963p transfected cells there is lower level of membrane-associated AREG than in scramble transfected cells, that is restored after ITGA6 overexpression (Fig. 40). Further, we treated primary human MF with 100 ng/mL AREG for 48h upon which we measured fibrogenic markers by qRT-PCR and immunocytochemistry. The expression of ACTA2, COL1A1, and S100A6 (Fig. 36 C-E ) and protein levels of SMA were upregulated upon AREG treatment, compared with the untreated cells, which indicates the significance of AREG-ITGA6 signalling in the activation of HSC (Figs. 38, 41, 42 and 43).

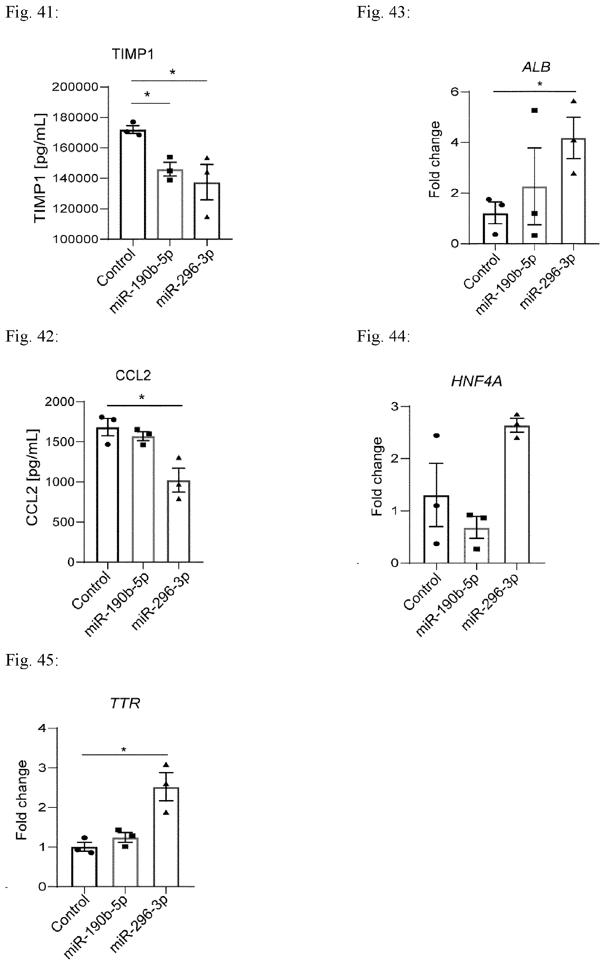

Overexpression of HAS2 and ITGA6 in myofibroblasts in vivo showed the importance of HAS2 and ITGA6 in liver fibrosis in vivo. ORFs encoding one of HAS2 and ITGA6 were cloned downstream of Pdgfrb promoter. AAV2p75-Pdgfrb-HAS2 contains the coding sequence for HAS2 under the control of the Pdgfrb promoter, and AAV2p75-Pdgfrb-ITGA6 contains the coding sequence for ITGA6 under the control of the Pdgfrb promoter. Additionally, viral vectors AAV2p75-Pdgfrb-190b-5p containing the coding sequence for 190b-5p under the control of the Pdgfrb promoter, AAV2p75-Pdgfrb-296-3p containing the coding sequence for 296-3p under the control of the Pdgfrb promoter, and as control AAV2p75-Pdgfrb-mCherry encoding the reporter mCherry under the control of the Pdgfrb promoter. First AAV2p75-Pdgfrb-190b-5p, AAV2p75-Pdgfrb296-3p or AAV2p75-Pdgfrb-mCherry was intravenously administrated into mice injected with CCl₄ for 6 weeks as schematically depicted in Fig. 44. Four days after initial AAV injections, one of AAV2p75-Pdgfrb-HAS2 or AAV2p75Pdgfrb-ITGA6 was administrated as a second injection to mice injected with AAV2p75-Pdgfrb-190b-5p or AAV2p75-Pdgfrb-296-3p, respectively. Ten days after the second AAV injections, mice were sacrificed and tissues were analysed.

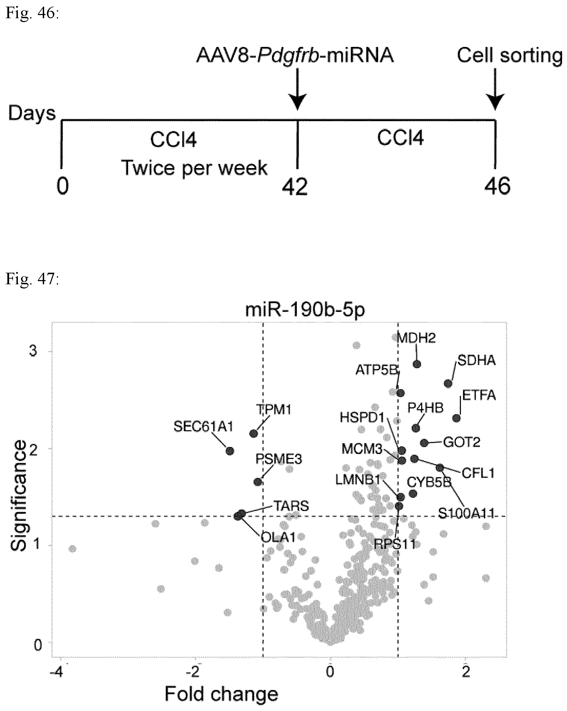

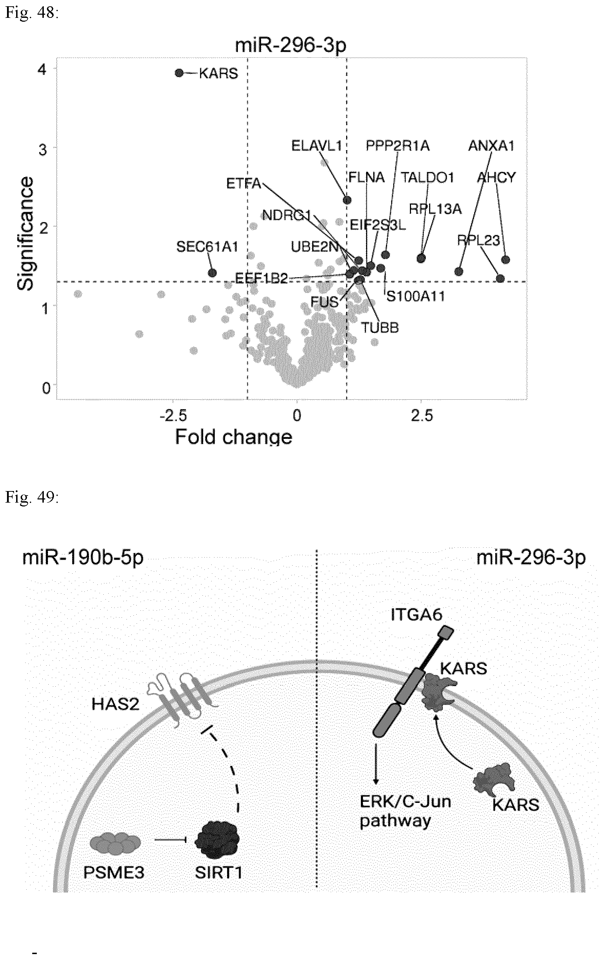

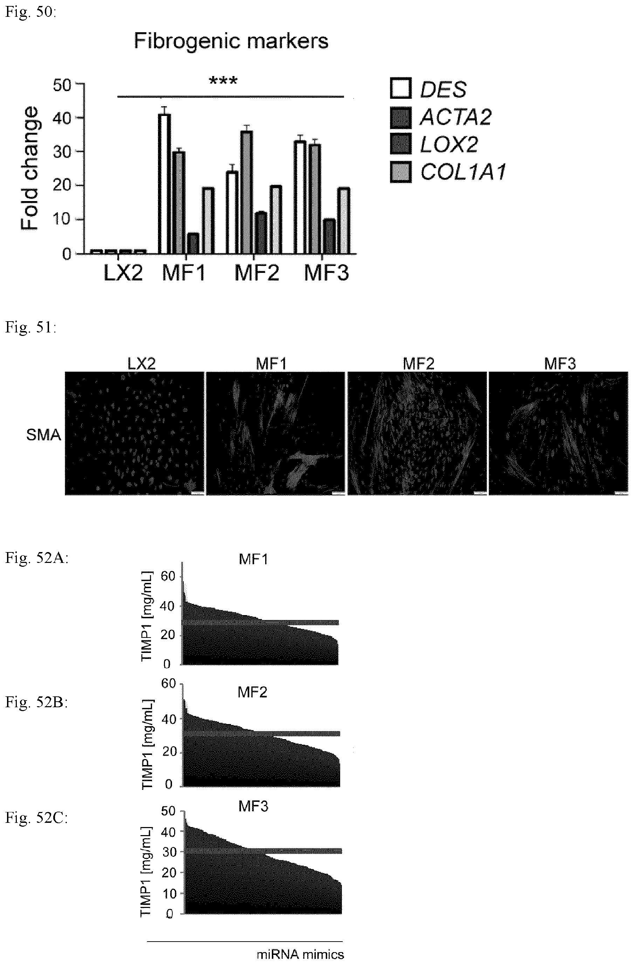

Overexpression of miR-190b-5p and miR296-3p was confirmed by qRT-PCR (Fig. 45). Then, the levels of Has2 and Itga6 were measured in the corresponding groups (Figs. 46, 47). The hydroxyproline content and the expression of fibrogenic markers Coll aland Acta2 (Figs. 48, 49) were determined. HE and Sirius Red staining, as well as SMA immunohistochemistry are shown in Fig. 50. Protein levels of HAS2 and ITGA6 were analysed by Western blotting and immunohistochemistry (Figs. 51, 52). Unfortunately, in each group, one mouse was an outlier. Notably, these were the mice with the lowest expression of HAS2 and ITGA6. On the basis of these data no significant differences in the expression of fibrogenic markers and in fibrosis degree was observed. In the ITGA6-overexpressing group significant histological changes were observed in the form of irregularly shaped cells, that seem to form and reside within their own niche, that is surrounded by collagen fibers.

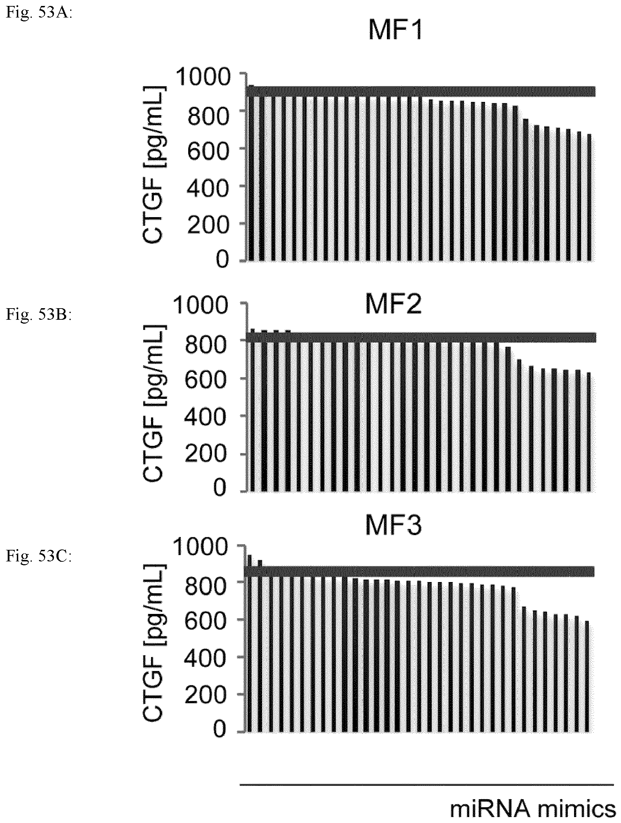

Human liver bud model confirmed that the inhibitors of HAS2 and ITGA6, miR-190b and miR-296, are suitable for use in the treatment and prevention of fibrosis. For generating the human liver bud model, primary human MF were transfected with miR-190b5p, miR-296-3p or Scramble. Transfected MF were then aggregated with primary human hepatocytes derived organoids, human Kupffer cells, and endothelial cells as schematically depicted in Fig. 53. 48h and 96h afterwards, the culture medium was collected in which TIMP1 levels (Fig. 54) and CCL2 levels by ELISA were measured. Consistent with the previous findings, lower levels of both TIMP1 and CCL2 were determined in liver buds in the presence of miR-190b and miR-296 than in the control (scramble).

Additionally, lower levels of a pro-inflammatory cytokine CCL2 in miR-190b-5p and miR-296-3p modulated liver buds we found, compared with the control. Furthermore, the expression of ALB (Fig. 56), HNF4A (Fig. 57), and TTR (Fig. 58) was measured by qRT-PCR. Notably, presence of miR-296-3p in liver buds resulted in significantly higher expression of ALB and TTR, compared with the control. This indicates that miR-296-3p might act as pro-hepatogenic miRNA as well. These results confirm that inhibitors of HAS2 and/or ITGA6, as exemplified by miR-190b-5p and miR-296-3p, are suitable as anti-fibrotic pharmaceutical compounds.

Protein changes that occur on the single cell level in vivo in myofibroblasts upon administration of AAV2p75-Pdgfrb-190b-5p or AAV2p75-Pdgfrb-296-3p were analysed by single cell proteomics (ScopeMS). At the 6th of CCl₄ injections in Lrat-Cre-ZsGreen mice, the AAVs we intravenously administrated as schematically depicted in Fig. 59. Four days afterwards, hepatocytes and all non-parenchymal cells were isolated, among which ZsGreen positive cells (HSC and HSC-derived MF) and ZsGreen negative cell populations were sorted by FACS. With ZsGreen positive cells, ScopeMs analyses were performed, and then comparing MS results of miRNA overexpressed ZsGreen+ MF with MS results of control injected ZsGreen+ MF. Five proteins were detected that were 2-fold downregulated in the AAV2p75-Pdgfrb-190b-5p group, compared with the control group (Fig. 60). Those proteins are TPM1, SEC61A1, PSME3, TARS and OLA1. Notably, TPM1 was previously reported to be upregulated on the surface of activated HSC and its expression correlates with the expression of SMA41. Moreover, after TGFβ1 treatment of MF, the expression of Tpml is induced, and it's necessary for the stable incorporation of SMA into the stress fibers, as schematically depicted in Fig. 62.

In the AAV2p75-Pdgfrb-296-3p group 2 proteins were detected that were 2-fold downregulated, compared with the control group (Fig. 61). Those two proteins are KARS and SEC61A1. In cancer cells KARS can translocate into the membrane and interact with ITGA6. The interaction results in the activation of ERK/C-Jun pathway and migration of cancer cells (Fig. 62). Of note, this pathway also contributes to the activation of HSC48.

On the example of AAV8-Pdgfrb-miRNA or AAV2p75-PdgfrbmiRNA these results also show that a miRNA which is an inhibitor of HAS2 and/or ITGA6 is preferably contained in a AAV-based viral particle, which has been shown to deliver the the anti-fibrotic miRNAs preferentially into myofibroblasts in vivo, e.g. in a pericentral and in a cholestatic model of liver fibrosis.

### Comparative Example: Testing antifibrotic activity of a large panel of miRNAs in human primary fibroblasts

As described in Example 1, primary human myofibroblasts (MF), and as a further comparison SMALX2 cells (LX2) were used for testing an antifibrotic activity of a number of microRNAs by analysing fibrogenic markers DES, ACTA2, LOX2 and CO1A1 by qPCR, measuring the modulation of TIMP1 secretion by ELISA, and measuring CTGF levels by ELISA. Analysis for these markers (Fig. 63) showed their significantly higher expression in MF than in LX2 cells. The pro-fibrogenic profile of the MF was confirmed by immunocytochemical staining for alpha smooth muscle actin (SMA) as shown in the micrograph of Fig. 64.

The microRNAs were used in the form of microRNA mimics, which are nonnatural doublestranded miRNA-like RNA fragments which at the 5'-end have a partially complementary motif to the 3'UTR of the target gene. Inside cells, this RNA fragment that mimics an endogenous miRNA binds to its target gene for inhibition of translation.

In this test, MF were used in three independent batches, and separate aliquots of MF were transfected with miRNA mimics. Fig. 65a, 65b, 65c show TIMP1 levels in three MF cultures MF1, MF2, MF3 in presence of miRNA mimics, with the horizontal line indicating the TIMP1 level in control (MF without miRNA mimic). The miRNAs that generated a high change of TIMP1 expression are identified in the following table:

**miRNA TIMP1 change (%)**

| | |
|---|---|
| hsa-miR-27b-3p | 52.94068 |
| hsa-miR-195-3p | 50.57661 |
| hsa-miR-18a-5p | 47.36129 |
| hsa-miR-487b-3p | 47.30115 |
| hsa-miR-486-3p | 47.28265 |
| hsa-miR-103a-2-5p | 34.80073 |
| hsa-miR-1247-5p | 34.23632 |
| **hsa-miR-190b-5p** | **31.95899** |
| hsa-miR-543 | 31.95205 |
| **hsa-miR-296-3p** | **30.49051** |
| hsa-miR-411-3p | 27.44713 |
| hsa-miR-423-3p | 25.9586 |
| hsa-miR-30c-1-3p | 22.13955 |
| hsa-miR-154-3p | 39.57282 |
| hsa-miR-433-5p | 35.1107 |
| hsa-miR-135b-5p | 31.96593 |
| hsa-miR-653-5p | 31.28316 |
| hsa-miR-200b-3p | 30.99324 |
| hsa-miR-145-5p | 30.8799 |
| hsa-miR-455-5p | 29.35782 |
| hsa-miR-199a-3p | 27.8797 |
| hsa-miR-183-3p | 25.72227 |
| hsa-miR-744-5p | 25.49828 |
| hsa-miR-301a-3p | 25.41848 |
| hsa-miR-9-3p | 24.70139 |
| hsa-miR-124-3p | 21.01303 |
| hsa-miR-744-3p | 20.23695 |
| hsa-miR-494-3p | 17.56292 |
| hsa-miR-129-1-3p | 12.87642 |
| hsa-miR-17-5p | 10.15265 |
| hsa-let-7g-5p | 25.0424 |
| hsa-miR-30b-5p | 28.0854 |
| hsa-miR-328-3p | 32.6469 |

These miRNAs, represented by their mimics, were then assayed for their effect in MF on CTGF expression levels. Fig. 53A-53C show expression levels of CTGF in separate aliquots of MF batches MF1, MF2, MF3, with the horizontal line indicating the CTGF level in control.

This test showed that of the miRNA panel, only miR-190b-5p and miR-296-3p, preferably in combination, have a strong anti-fibrogenic effect.

## Claims

1. Compound for use in the prevention or treatment of liver fibrosis, **characterized in that** the compound is miR-190b-5p, miR-296-3p, or a combination of these.

2. Compound for use in medical treatment or prevention, **characterized in that** the compound is an inhibitor of hyaluronan synthetase (HAS2) and/or an inhibitor of integrin alpha-6 (ITGA6).

3. Compound for use in medical treatment or prevention according to claim 2, wherein the medical treatment or prevention is the treatment or prevention of liver fibrosis.

4. Compound for use according to one of claims 2 to 3, **characterized in that** the inhibitor of hyaluronan synthetase (HAS2) is miR-190b-5p.

5. Compound for use according to one of claims 2 to 4, **characterized in that** the inhibitor of integrin alpha-6 (ITGA6) is miR-296-3p.

6. Compound for use according to one of the preceding claims, **characterized in that** the miR-190b-5p and/or miR-296-3p is encoded in an expression cassette under the control of a promoter that is specifically expressed in pro-fibrotic myofibroblast cells (MF).

7. Compound for use according to claim 6, **characterized in that** the promoter that is specifically expressed in pro-fibrotic myofibroblast cells is the Pdgfrb promoter.

8. Compound for use according to one of the preceding claims, **characterized in that** the miR-190b-5p, miR-296-3p, or a combination of these is encoded in an AAV-based viral particle.

9. Compound for use according to claim 3, **characterized in that** the inhibitor of hyaluronan synthetase (HAS2) is at least one of 4-methylumbelliferone (4-MU or MU), 4-methylesculetin (ME), brefeldin A.

10. Compound for use according to one of the preceding claims, **characterized in that** liver fibrosis is periportal and/or pericentral and/or cholestatic liver fibrosis.
